(19)

Europäisches Patentamt
European Patent Office
Office européen des brevets

(11) **EP 2 535 001 A1**

(12) **EUROPEAN PATENT APPLICATION**

(43) Date of publication:
**19.12.2012 Bulletin 2012/51**

(51) Int Cl.:
**A61B 6/00** *(2006.01)*     **G06T 7/00** *(2006.01)*

(21) Application number: **11169814.8**

(22) Date of filing: **14.06.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Radiology Morphological Solutions B.V.**
**3016 CM Rotterdam (NL)**

(72) Inventor: **Mended, Richard**
**3016 CM Rotterdam (NL)**

(74) Representative: **Jansen, Cornelis Marinus et al**
**VEREENIGDE**
**Johan de Wittlaan 7**
**2517 JR Den Haag (NL)**

(54) **Method, a system and a computer program product for registration and identification of diagnostic images**

(57)     The invention relates to a method for registration of diagnostic images taken for different time moments, comprising the steps of retrieving an earlier diagnostic image, retrieving a later diagnostic image, registering the earlier diagnostic image and the later diagnostic image, identifying differences between the earlier diagnostic image and the later diagnostic image, marking an area corresponding to a difference between the earlier and the later diagnostic image on the later diagnostic image. The invention further relates to a system and a computer program for registration of diagnostic images taken for different time moments.

Fig. 1

**Description**

Field of the invention

The invention relates to a method for registration of diagnostic images taken at different time moments for the same patient.

The invention further relates to a system for registration of diagnostic images taken at different time moments for the same patient.

The invention still further relates to a computer program product for registration of diagnostic images taken for different time moments for the same patient.

Background of the invention

In medical practice quite often a patient is being examined for purposes of follow-up examination at different times using substantially the same field of view. For example, for thorax examinations, transmission X-ray images of lungs and surrounding tissue, such as ribs, may be taken.

It is a common practice for the radiologists to review the suitable images taken at different moments, further on referred to as the earlier diagnostic images and the later diagnostic images using light tables or using computer displays, should the images be digitized.

However, it is found to be quite challenging to carry out accurate assessment of the possible differences between the earlier diagnostic images and the later diagnostic images for a number of reasons. First, the field of view taken for these images may be different. Secondly, an image may be (slightly) distorted due to organ or patient displacement. Thirdly, a plurality of different diagnostic apparatus having different operational settings may be used for acquiring the diagnostic images under consideration. Fourth, the patient may have changed dimensions, because of age, operation or due to a weight change. And finally, human errors may occur during visual inspection of the images.

Summary of the invention

It is an object of the invention to provide a method for registration of diagnostic images taken for different time moments for the same patient, which is accurate and which allows for quantification of the differences between the earlier and the later diagnostic image.

To this end the method according to the invention comprises the steps of:

- retrieving an earlier diagnostic image;
- retrieving a later diagnostic image;
- automatically registering the earlier diagnostic image and the later diagnostic image;
- automatically identifying differences between the earlier diagnostic image and the later diagnostic image;
- automatically marking an area corresponding to a difference between the earlier and the later diagnostic image on the later diagnostic image.

It is found that with a suitable image processing, drawbacks of the prior art may be mitigated to a substantial extent. For example, when the step of image registration is carried out, misalignments and distortion artefacts can be eliminated. It will be appreciated that any suitable image registration method is suitable for practicing the invention. It will be appreciated that although the invention may be carried out using two-dimensional X-ray transmission images, it is applicable to other imaging modalities and more dimensional images, such as 3D, 4D or 5D images. Those skilled in the art will readily appreciate which three-dimensional image registration algorithms may be applied for the 3D images.

In an embodiment of the invention the step of registering the earlier diagnostic image and the later diagnostic image comprises the steps of:

- identifying a set of reference marks in the earlier diagnostic image and the later diagnostic image;
- superpositioning the earlier diagnostic image and the later diagnostic image by using the at least one reference mark.

Preferably, the superpositioning is carried out using identified locations of the static reference points in the image. For example, specific structures or regions, such as lung top, lung border, bony structures, such as ribs may be used for that purpose. Those skilled in the art will readily appreciate which per se known algorithms may be used for enabling automatic delineation of the bony structures, such as ribs, in the diagnostic images under consideration.

When the earlier image and the later diagnostic image are registered, image intensities may be subtracted yielding the corresponding sought difference. This difference may be used as an aid for making a diagnosis.

It is found that in order to distinguish relevant medical differences from signals caused by noise and/or misplacements in the superpositioning process a correlation algorithm may be applied on the found differences after matching and subtraction between the earlier diagnostic image and the later diagnostic image has been made.

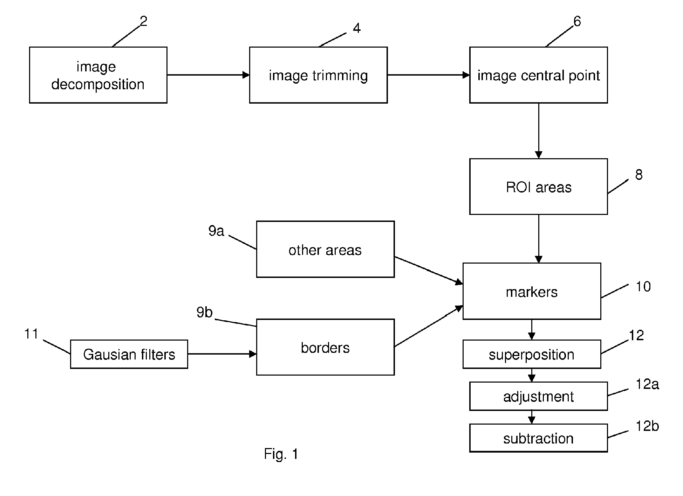

In a still further embodiment of the method according to the invention, the method further comprises a step of applying a filter to the earlier diagnostic image and/or the later diagnostic image for further post-processing of the images. This embodiment is explained in further detail with reference to Figure 1.

In a still further embodiment of the method according to the invention the method further comprises the step of adjusting the image contrast prior to image registration.

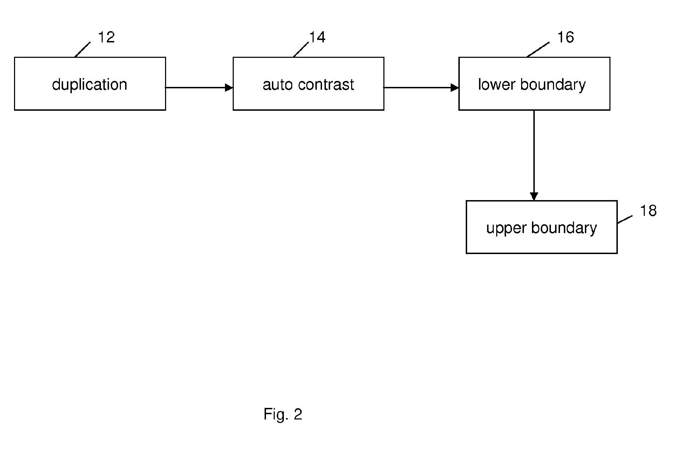

The contrast may be adjusted locally or dynamically. It is found to be particularly advantageous to carry out contrast adjustment of the diagnostic images under consideration, for example to eliminate influence of accidental bright areas surrounding the actual image. This step is found advantageous because contrast adjustments may affect the identification of very small/low intensity differences between the images. This embodiment will be explained in more detail with reference to Figure 2.

In a still further embodiment of the method according to the invention it further comprises the steps of:

- identification of a region of interest in the earlier diagnostic image and in the later diagnostic image;
- elimination of structures from the earlier diagnostic image and the later diagnostic image not corresponding to the said set of markers and the region of interest.

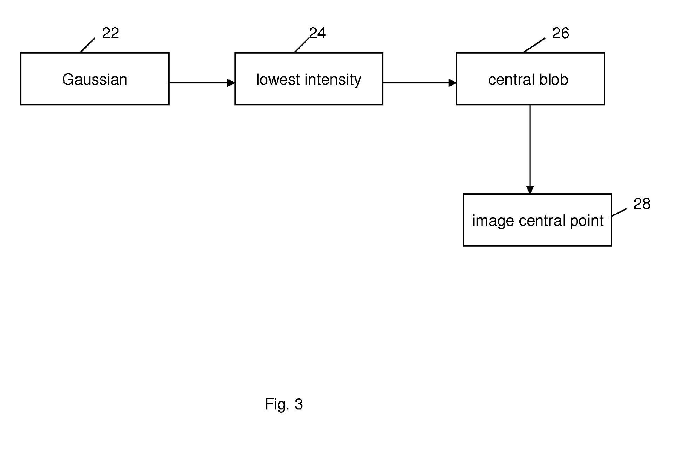

It is found that by eliminating the structures not part of the image of interest data processing may be further improved and accelerated. More details on this particular embodiment will be given with reference to Figure 3.

In a still further embodiment of the method according to the invention, the method further comprises the step of visualization of the area corresponding to a difference between the earlier and the later diagnostic image.

Preferably, the detected differences are quantified and color coded for indicating the suspected medical relevance. More preferably, an image representing the color coded differences is superimposed on the later diagnostic image.

The system for registration of diagnostic images taken for different time moments, comprises:

- a processor adapted to :

    i. retrieving an earlier diagnostic image;
    ii. retrieving a later diagnostic image;
    iii. automatically registering the earlier diagnostic image and the later diagnostic image;
    iv. automatically identifying differences between the earlier diagnostic image and the later diagnostic image;
    v. automatically marking an area corresponding to a difference between the earlier and the later diagnostic image on the later diagnostic image.

In an embodiment of the system according to the invention it further comprises a display arranged to display the earlier diagnostic image, the later diagnostic image and the area or areas corresponding to a difference between the earlier diagnostic image and the later diagnostic image superimposed on the later diagnostic image.

The computer program product for registration of diagnostic images according to the invention comprises instructions for causing a processor to carry out the steps of the method as is discussed in the foregoing.

These and other aspects of the invention will be explained in more detail with reference to figures. In the figures like elements are depicted using like reference signs. It will be appreciated that the figures are given for illustrative purposes only and may not be used for limiting the scope of the appended claims.

Brief description of the drawings

Figure 1 presents in a schematic way an embodiment of a method according to the invention.
Figure 2 presents in a schematic way an embodiment of a contrast adjustment step.
Figure 3 presents in a schematic way an embodiment of a region of interest identification step.
Figure 4 presents in a schematic way an embodiment of a further embodiment of a region of interest identification step.
Figure 5 presents an embodiment of an earlier diagnostic image.
Figure 6 presents an embodiment of a later diagnostic image.
Figure 7 presents an embodiment of a diagnostic image comprising color coded data on a difference between the earlier diagnostic image and the later diagnostic image.

Detailed description of the drawings

Figure 1 presents in a schematic way an embodiment of a method according to the invention. It will be appreciated that the given sequence of steps is not exhaustive, nor binding.

In accordance with an embodiment of the method of the invention a pyramidal image decomposition may be carried out at step 2. For example, for each analyzed image a lowpass pyramid may be generated, for example by first smoothing the image with an appropriate smoothing filter and then subsampling the smoothed image by a factor of two along each coordinate direction. As this process proceeds, the result will be a set of gradually more smoothed images, wherein in addition the spatial sampling density is decreasing.

The lowpass filter may be realized with a two dimensional convolution, implemented using two successive one dimensional convolutions along the rows and columns of the image.

At step 4 suitable image processing (trimming) may take place. This step will be explained in more detail with reference to Figure 2.

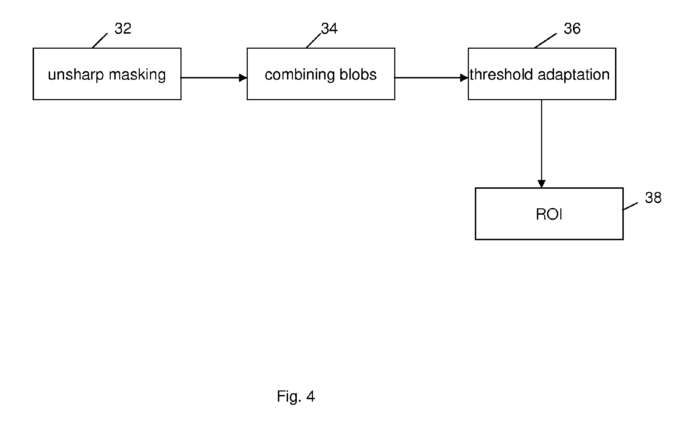

At step 6 the image is suitably processed, an image central point may be established. This step is explained in more detail with reference to Figure 3. Next, suitable identification of the region of interest (ROI) may be carried out at step 8. Those skilled in the art will readily appreciate which per se known method may be used for this purpose. More particular details on the ROI identification step are given with reference to Figure 4. Afterwards, at step 10 suitable landmarks may be identified. In case when the image comprising bony structures it may be preferable to use bones as suitable markers for purposes of image registration between the earlier diagnostic image and the later diagnostic image. However, other structures or organs may be used for this purpose as well. For example, for identification of markers use may be made of delineation of other areas and borders at the respective steps 9a, 9b. The borders may be obtained using knowledge on the expected structures in the diagnostic image. For example, for thorax images, ribs may be delineated using Gaussian filters 11.

After the set of markers is established at step 10, the method according to the invention proceeds to steps of superpositioning of the earlier diagnostic image with the later diagnostic image at step 12, adjustment of the images, notably the regions of interest, at step 12a and subtraction of data in the images (or regions of interest) at step 12b.

Figure 2 presents in a schematic way an embodiment of a contrast adjustment step. It is found that during the processing of diagnostic images, such as radiographs, bright lines may appear in areas surrounding the actual image and cause undesirable interference with the feature detection algorithm used for detection of patterns in the diagnostic image under consideration. The exemplary steps 12 - 18 may be applied to mitigate this problem.

First, at step 12 duplication of low resolution image may be carried out. At step 14 a suitable auto contrast algorithm may be applied. The auto contrast procedure expands the dynamic range of the image intensity by mapping the lightest and darkest pixels to maximum (for example, 255) and minimum (0) possible values. To determine the lightest and darkest pixels, histogram clipping may be used. The maximum value of the image intensity (lightest pixels) may be determined so that only 0.5% of the image pixels have the intensity higher than the maximum. For the minimum the same levels are applied, only 0.5% of the pixels have the intensity lower than the maximum. It will be appreciated, however, that any pre-determined value may be used for setting the respective lower and upper cut-off maximum/ minimum intensities.

Next, at step 16 a search may be carried out for the first 20% of the image from each side; top, bottom, left and right. The first row with a mean intensity value above 10 may be treated as signal level, the respective boundary of the image. All rows towards the image border may be duplicated with the intensity values found in this row.

At step 18 the upper boundary may be set. For the top boundary additional steps may be performed. The row with the minimum intensity is searched within the predetermined level, say 5% of the image. All rows located above this minimum intensity row may be normalized by using $I(x) = I(x) S_{min}/S$, wherein $I(x)$ refers to the intensity of the pixel at location x. $S_{min}$ refers to the intensity sum of found minimum row and S refers to the sum of all pixel intensities of current row.

Figure 3 presents in a schematic way an embodiment of a region of interest identification step. Thorax X-ray examination may contain a great number of informational structures. The region of interest (ROI) for thorax are lung areas. It is found that for enabling accurate assessment of lung areas it may be preferable to eliminate other structures from the image, except for markers used for image registration.

In this exemplary embodiment at step 22 the Gaussian of image A may be calculated as the convolutions of image intensity $I(x,y)$ with the Gaussian $2^{nd}$ kernel function. Preferably sigma = 25 is used yielding G(25).

At step 24 the lowest intensity is determined. For this purpose the Gaussian(25) may be divided into two equal sides. For each side a search may be carried out on the top half of the image with the exclusion of a predetermined portion (20% for example) of the image boundaries for the lowest intensity signal of $G > G_{minLeft}$, GminRight.

At step 26 an image region may be calculated. An image region is a connected image area containing pixels with the same close value of intensity. A list of image regions may be calculated using the intensity $G_{minLeft}$, $G_{minRight}$ for both sides of image G(25). Regions with width and height more than ¼ of image size may be excluded from the list. In each side of image G(25) one may find a central region which is closest to the center of the image. The central region may be used to determine coordinates of centre of both lungs in a thorax image, ($X_{left}$, $Y_{left}$) and ($X_{right}$, $Y_{right}$) as

$$X = (X_{min}+X_{max})/2; \; Y = (Y_{min}+Y_{max})/2.$$

$X_{min}$ - minimum horizontal coordinate of pixels from central image region;
$X_{max}$ - maximum horizontal coordinate of pixels from central image region;
$Y_{min}$ - minimum vertical coordinate of pixels from central image region;

$Y_{max}$ - maximum vertical coordinate of pixels from central image region.

It will be appreciated that this example is given for one side of the lungs of a thorax photo taken as an exemplary embodiment of the diagnostic image. In practice the calculation may be done for both sides.

At step 28 a central point may be identified. For example, thoracic central point may be calculated as follows:

$$X_{center} = (X_{left} + X_{right})/2$$

$$Y_{center} = (Y_{left} + Y_{right})/2$$

Next, identification of lung areas may be carried out, which is described with reference to Figure 4.

Figure 4 presents in a schematic way an embodiment of a further embodiment of a region of interest identification step. The identification of the region of interest (ROI) may be done using Gaussian filters. This technique results in a clear separation between the ROI and structures outside this region. The image region corresponding to the lung area and it's bounding rectangle may be stored.

At step 32 unsharp masking may be carried out. For the unsharp masking algorithm a Gaussian G(3) of image A may be formed with sigma 3, for example, a Gaussian G(40) of image G(3) may be calculated with sigma 40. It will be appreciated that different values for sigma may be used as well. An unsharp mask image may be calculated using G(3) and G(40) with an amplification factor of 5. It will be appreciated that these examples are not limiting.

At step 34 combining of lung regions is carried out. The Gaussian filter used at step 32 results in a clear separation between the lung and outside area. Within the lung area the collarbone may cause a complete region separation between the top part of the bottom part of the lung. In order to achieve 1 image region of the total ROI for the left and right side lines may be drawn from the weighted center upwards with the following restrictions and settings:

- lines drawn with a 3 pixel width;
- dl line is drawn from the weighted centre coordinate till height coordinate image hc/7;
- dl length is restricted to distance between the weighted centre and dc/2;
- drawn lines in left and right side are inclined to centre of image by dl/8.

At step 36 a threshold adaptation algorithm may be applied/ Adaptation may be done iteratively with thresholds decreasing from 50 till 27 with steps of 3 in intensity levels. However, other values may be used. For each step a binary image image region may be constructed from USM threshold.

The image regions created during the threshold adaptation algorithm may be tested against the following rules:

- outer positions of image region are not less than a predetermined number of pixels (for example, 5) away from the image boundary;
- $X_{left}$, $Y_{left}$, $X_{right}$, $R_{right}$ coordinates are located inside the image region;
- X and Y coordinates of top most pixel for both sides may be annotated as lung top coordinates (ptLeft, ptRight) he distance between $X_{center}$ and x coordinate for lung top is not allowed to be greater than the distance between $X_{center}$ and $_{Xleft}$;
- Difference in Y coordinates of lung top coordinates is not allowed to be greater than distance/const, for example const = 10.

It will be appreciated that different approaches for implementing testing may be used.

At step 38 all the above requirements are checked. Should they be met, the image region is treated as the ROI area. If only one region is found after adaptation iterations it is excluded and mirroring of coordinates from other side around $X_{center}$ is used. It will be appreciated that use can be made of suitable structures, such as lung top, lung border or the like.

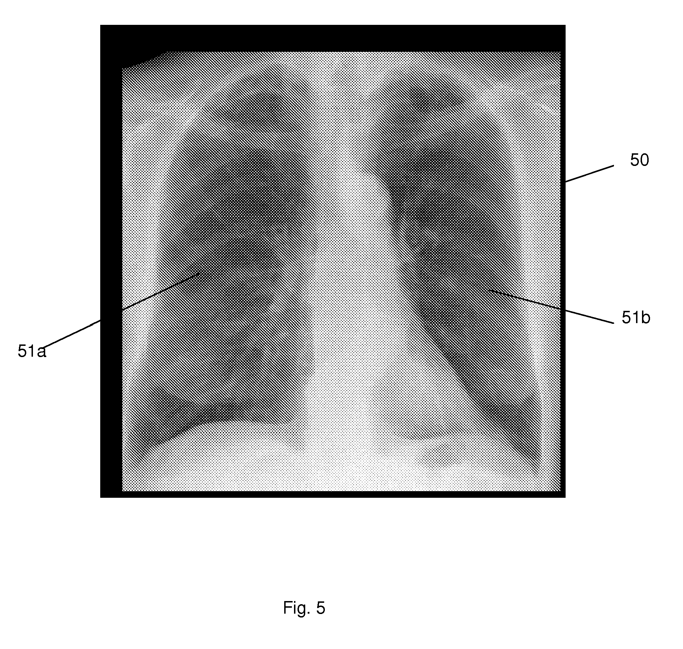

Figure 5 presents an embodiment of an earlier diagnostic image. In this particular embodiment a thorax image is used. In the thorax image 50 the relevant structures may relate lung structures 51a and 51b.

When the earlier diagnostic image and the later diagnostic image are processed as described above, the images are registered. However, it may be necessary to carry out the step of image registration prior to image warping. Because relevant differences are to be investigated with respect to the present state of the patient, it is found advantageous to warp the earlier diagnostic image to the later unrectified diagnostic image.

The procedure of image warping is known per se and may comprise the following steps:

- checking and adjustment of the feature detection points (markers);
- normalization of point coordinates;
- estimation of widths of specific structures, such as bony structures (ribs);
- calculation of a central point;
- selection/normalization of points in the surroundings (ribcage points);
- labeling;
- filtering for displacement;
- correlation refinement for motion correction;
- recalculation of reference points in the surroundings (rib cage points);
- calculation of displacement quality measures;
- warping the image.

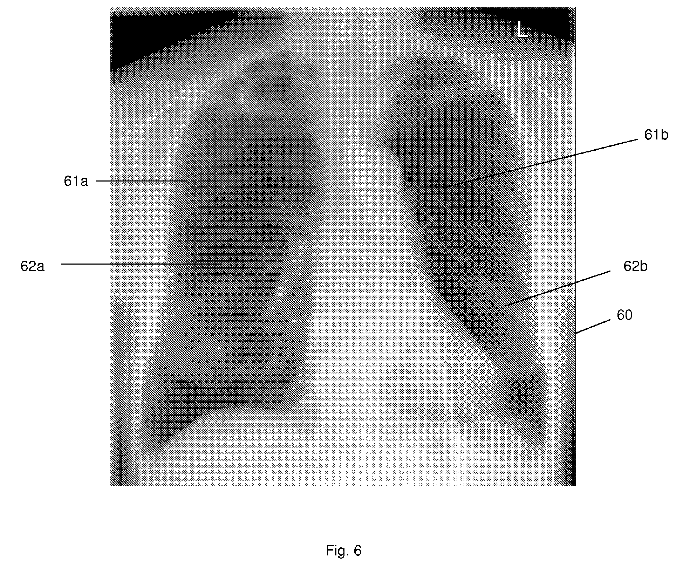

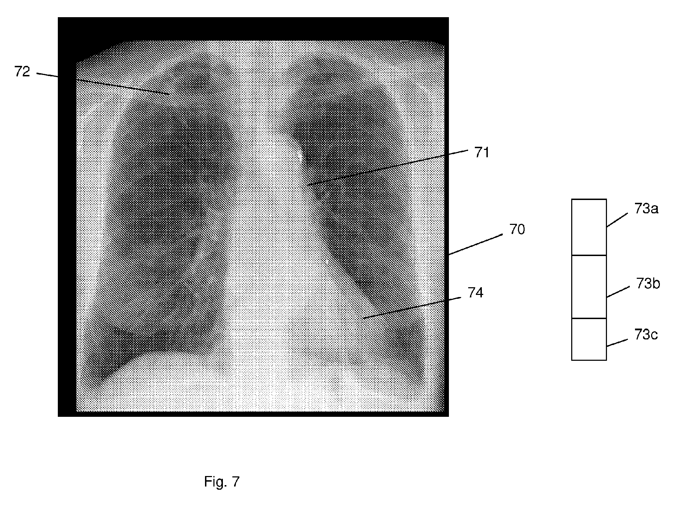

Figure 6 presents an embodiment of a later diagnostic image 60. In this image the relevant structures are the ribs 62a, 62b and the lung 61a, 61b. Figure 7 presents an embodiment of a later diagnostic image 70 comprising color coded data 71, 72, 74 representing the difference data, overlaid on the later diagnostic image. For the convenience, the image may be appended with a suitable legend comprising segments 73a, 73b, 73c. It will be appreciated that the difference image may comprise positive areas and negative areas. Interpretation of the difference data may be dependent upon a clinical case.

It is found that provision of such quantified overlaid image comprising accurate information on the difference in volumes of regions of interest of the same patient over time has substantial added value. It will be further appreciated that because the invention is based on analysis of actual images its results are more robust than results obtained using the methods known in the art, such as applications based on pattern recognition and modeling.

It will be appreciated that the invention is not limited to the embodiments which have been just described. It will be appreciated that the invention may be practiced otherwise than as described.

**Claims**

1. Method for registration of diagnostic images taken for different time moments, comprising the steps of:

   - retrieving an earlier diagnostic image;
   - retrieving a later diagnostic image;
   - automatically registering the earlier diagnostic image and the later diagnostic image;
   - automatically identifying differences between the earlier diagnostic image and the later diagnostic image;
   - automatically marking an area corresponding to a difference between the earlier and the later diagnostic image in the later diagnostic image.

2. The method according to claim 1, wherein the step of automatically registering the earlier diagnostic image and the later diagnostic image comprises the steps of:

   - identifying a set of reference marks in the earlier diagnostic image and the later diagnostic image;
   - superpositioning the earlier diagnostic image and the later diagnostic image by using a set of reference marks.

3. The method according to claim 2, wherein the step of automatically identifying differences between the earlier diagnostic image and the later diagnostic image comprises the step of:

   - transformation of the earlier diagnostic image;
   - subtraction of intensity signals between the registered earlier diagnostic image and the later diagnostic image.

4. The method according to any one of the preceding claims, further comprising a step of applying a filter to the earlier diagnostic image and/or the later diagnostic image for post-processing of the images.

5. The method according to claim 4, wherein the filter comprises a low pass smoothing filter based on data convolution.

6. The method according to any one of the preceding claims, further comprising the step of adjusting the image contrast prior to image registration.

7. The method according to any one of the preceding claims, wherein the earlier diagnostic image and the later diagnostic image comprise an image of a displaceable organ.

8. The method according to claim 7, wherein the earlier diagnostic image and the later diagnostic image comprise a thorax image.

9. The method according to any one of the preceding claims, wherein for the at least one reference mark a portion of a bone is selected.

10. The method according to claim 8 or 9, further comprising the steps of:

   - identification of a region of interest in the earlier diagnostic image and in the later diagnostic image;
   - elimination of structures from the earlier diagnostic image and the later diagnostic image not corresponding to the said of markers and the region of interest.

11. The method according to any one of the preceding claims, further comprising the step of visualization of the area corresponding to a difference between the earlier and the later diagnostic image.

12. The method according to claim 11, wherein said visualization is carried out using a color code.

13. System and a computer program product for registration of diagnostic images taken for different time moments, comprising:

   - a processor adapted to :

      i. retrieving an earlier diagnostic image;
      ii. retrieving a later diagnostic image;
      iii. automatically registering the earlier diagnostic image and the later diagnostic image;
      iv. automatically identifying differences between the earlier diagnostic image and the later diagnostic image;
      v. automatically marking an area corresponding to a difference between the earlier and the later diagnostic image on the later diagnostic image.

14. The system according to claim 13, further comprising a display arranged to display the earlier diagnostic image, the later diagnostic image and the area corresponding to a difference between the earlier diagnostic image and the later diagnostic image

15. Computer program product for registration of diagnostic images comprising instructions for causing a processor to carry out the steps of the method as is claimed in claims 1 - 12.

Fig. 1

Fig. 2

22

Gaussian

24

lowest intensity

26

central blob

28

image central point

Fig. 3

```
    ┌─ 32                    ┌─ 34                    ┌─ 36
┌───────────────┐       ┌───────────────┐       ┌─────────────────────┐
│unsharp masking│──────▶│combining blobs│──────▶│threshold adaptation │
└───────────────┘       └───────────────┘       └─────────────────────┘
                                                            │
                                                            ▼
                                                 ┌─────────────────┐─ 38
                                                 │      ROI        │
                                                 └─────────────────┘
```

Fig. 4

50

51b

51a

Fig. 5

Fig. 6

Fig. 7

Europäisches
Patentamt

European
Patent Office

Office européen
des brevets

# EUROPEAN SEARCH REPORT

Application Number

EP 11 16 9814

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2003/018245 A1 (KAUFMAN LEON [US] ET AL) 23 January 2003 (2003-01-23)<br>* abstract *<br>* paragraphs [0002], [0006], [0009] - [0019], [0039] - [0044], [0047] - [0048], [0050] - [0052], [0060], [0062] - [0075], [0137], [0139]; claims 1,2; figures 1-3,4,5-6 * | 1-3,6-8, 10-15<br><br>9 | INV.<br>A61B6/00<br>G06T7/00 |
| X<br><br>A | WO 99/05641 A1 (ARCH DEV CORP [US]) 4 February 1999 (1999-02-04)<br>* abstract *<br>* page 3, line 23 - page 4, line 10 *<br>* page 7, line 10 - page 13, line 8; figures 1a-c * | 1-8,10, 13<br><br>9 | |
| X | WO 2006/054194 A2 (KONINKL PHILIPS ELECTRONICS NV [NL]; PEKAR VLADIMIR [DE]; KAUS MICHAEL) 26 May 2006 (2006-05-26)<br>* page 2, line 22 - line 27 *<br>* page 5, line 15 - page 6, line 7 *<br>* page 8, line 14 - line 31; figure 4 * | 1,2,9,13 | |
| | | | TECHNICAL FIELDS SEARCHED (IPC) |
| X<br><br>Y | US 2008/317314 A1 (SCHWARTZ LAWRENCE H [US] ET AL) 25 December 2008 (2008-12-25)<br>* paragraphs [0019], [0052] - [0058], [0062], [0068] - [0070], [0072] - [0079]; figures 1a,2a,3,4,17 * | 1,13<br><br>9 | A61B<br>G06T |
| X<br><br>A | US 2008/118132 A1 (UBELHART ISTVAN [HU] ET AL) 22 May 2008 (2008-05-22)<br>* paragraphs [0001], [0029] - [0033], [0037], [0039]; figures 3,4,5,6 * | 1,13<br><br>4,5,7,9 | |

The present search report has been drawn up for all claims

| Place of search | Date of completion of the search | Examiner |
|---|---|---|
| The Hague | 14 November 2011 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 16 9814

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2011

| Patent document cited in search report | | | Publication date | Patent family member(s) | | | Publication date |
|---|---|---|---|---|---|---|---|
| US 2003018245 | A1 | | 23-01-2003 | US | 2003018245 | A1 | 23-01-2003 |
| | | | | US | 2005251021 | A1 | 10-11-2005 |
| WO 9905641 | A1 | | 04-02-1999 | AU | 8579898 | A | 16-02-1999 |
| | | | | EP | 0998721 | A1 | 10-05-2000 |
| | | | | JP | 4718003 | B2 | 06-07-2011 |
| | | | | JP | 2001511570 | A | 14-08-2001 |
| | | | | US | 5982915 | A | 09-11-1999 |
| | | | | WO | 9905641 | A1 | 04-02-1999 |
| WO 2006054194 | A2 | | 26-05-2006 | CN | 101061520 | A | 24-10-2007 |
| | | | | EP | 1817744 | A2 | 15-08-2007 |
| | | | | JP | 2008526270 | A | 24-07-2008 |
| | | | | US | 2009074264 | A1 | 19-03-2009 |
| | | | | WO | 2006054194 | A2 | 26-05-2006 |
| US 2008317314 | A1 | | 25-12-2008 | NONE | | | |
| US 2008118132 | A1 | | 22-05-2008 | DE | 102007057096 | A1 | 29-05-2008 |
| | | | | US | 2008118132 | A1 | 22-05-2008 |

EPO FORM P0459

For more details about this annex : see Official Journal of the European Patent Office, No. 12/82